# EUROPEAN PATENT APPLICATION

(11) **EP 4 813 420 A1**
(43) Date of publication of application: **30.09.2026**
(21) Application number: 25166404.1
(22) Date of filing: 26.03.2025
(51) Int. Cl.: A61M 5/20, A61M 5/315, A61M 5/31

(54) **END OF INJECTION SIGNAL FOR AN INJECTION DEVICE**

(71) Applicant: Ypsomed AG, 3401 Burgdorf (CH)
(72) Inventor: Hostettler, Patrick, 3415 Hasle (CH); Schenker, Susanne, 4912 Aarwangen (CH); von Mühlenen, Beat, 3043 Uetligen (CH); Vogler, Roman, 3097 Liebefeld (CH); Wachter, René, 3063 Ittigen (CH)
(74) Representative: Kleiner, Stefan

(57) **Abstract**

The invention relates to an end of injection signalling mechanism (20) for an injection device (1) for dispensing a liquid drug through an injection needle. The signalling mechanism (20) comprises a housing (2), a rotation member (3) and a flexible signal member (22) adapted to generate an audible or tactile end of injection signal only at the end of an injection. The signal member (22) can be released from a tensioned state by a rotation of the rotation member (3) to generate the signal. The signalling mechanism (20) further includes a tensioning member (30) adapted deform the signal member (22) wherein the signal member (22) can be deformed and tensioned by a relative tensioning movement between the tensioning member (30) and the signal member (22) exclusive along the longitudinal axis without relative rotation.

## Description

### FIELD OF THE INVENTION

The present invention relates to injection devices or medicament delivery devices for injecting, delivering, administering, infusing or dispensing substances and/or liquids such as insulin or hormone preparations. It departs from an end of injection signaling mechanism for an injection device. The signaling mechanism comprises a housing, a rotation member rotatable relative to the housing during dispensing of a dose and a flexible signal member adapted to be released by the rotation member to generate an audible or tactile signal only at the end of the injection.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

EP 1877119 B1 discloses an injection pen with a mechanism that provides non-visual feedback to the user during dose setting and injection. A click member, during injection, produces a clicking sound as its teeth engage with a ratchet tooth. This sound occurs when the set dose is almost fully injected, providing auditory feedback to the user. In further embodiments a mechanism is disclosed for tensioning and releasing the spring arm, utilizing rotating parts and wedge structures to generate tactile and audible feedback signals when the injection is completed.

EP3003434 B1 discloses an injection pen including a scale drum that is movable in a helical manner during dose setting and during injection. A click arm is tightened when the scale drum rotates toward a zero position, creating an audible click to notify the user that the dose has been administered. When the scale drum rotates back during injection, it engages the click arm with a distally pointing protrusion.

EP3041538 B1 discloses an injection device including an end-of-dose signal mechanism. As a spring releases its stored energy, a scale drum rotates back toward an initial position. As the rotating scale drum approaches a stop position, a flexible click arm rides over an inner housing flexible arm and creates an audible click to signal the user that the injection is complete.

### DESCRIPTION OF THE INVENTION

It is an objective of the invention to provide a simple and yet reliable mechanism for indicating an end of the injection to the user.

This objective is achieved by an end of injection signaling mechanism and a method according to the independent claims. Preferred embodiments are evident from the dependent claims.

The invention relates to an end of injection signaling mechanism for an injection device for dispensing a dose of liquid drug through an injection needle. The signaling mechanism comprises
- a housing defining a longitudinal axis and enclosing a dose and dispensing mechanism of the injection device;
- a rotation member rotatable relative to the housing at least during dispensing of a dose towards an end position in which the dose is completely dispensed and in which the rotation member abuts and end stop;
- a flexible signal member movable or deflectable relative to the housing and adapted to generate an audible or tactile end of injection signal only at the end of the dose dispensing (end of injection), for example, a click noise to the user.

The signal member can be released from a tensioned state by the rotation member or an intermediate member coupled to the rotation member into a release state to generate the end of injection signal.

Before the injection ends, the signal member can be brought or moved from the released state into the tensioned state by a relative tensioning movement between a tensioning member and the signal member. That means the signal member and the tensioning member hit or contact each other. The signal member is deformed by the relative tensioning movement. The tensioning movement is exclusively along the longitudinal axis without rotation relative to the housing or relative to any other component.

That means the flexible signal member moves relative to the tensioning member or the tensioning member moves relative to the click member to tension or prestress the flexible signal member into the tensioned state to prepare the signal member for generating the end of injection signal.

The pure axial relative tensioning movement allows for a simple and reliable tensioning of the flexible signal member to prepare the signal member for the generation of the end of injection signal. As the signal member is tensioned by an exclusive longitudinal movement the function of tensioning can be implemented by longitudinally moving components of the existing dose and dispensing mechanism of the injection pen, for example, by a longitudinal movement of a push button or actuation sleeve.

Hence, contrary to prior art approaches the signal member is not tensioned or prestressed by a rotation sleeve, e.g. a number sleeve or scale drum. According to the present invention the signal member is tensioned by an exclusive longitudinally acting component of the mechanism. The longitudinal movement may be directly linked to a release or actuation movement to initiate the injection and hence it is ensured that the signal member is actually tensioned before the end of injection. Moreover, as the rotation member is usually drive spring actuated, the tensioning movement is independent of the drive spring. The longitudinal tensioning movement for the signal member is at least an alternative to the known rotational tensioning approaches.

The rotation member may be any rotating component, and in particular, it may be a component of a dose and dispensing mechanism of the injection device. Hence, the rotation member may be used exclusively in the signaling mechanism or, more preferably, the rotation member may be an existing component of a dose or dispensing mechanism of the injection device. By way of example, the rotation member may be a number sleeve, a drive member or a sleeve or barrel that rotates at least during dose dispensing.

In the case of a manual injection device the rotation member may be driven by user force and for an automatic injection device the rotation member may be rotated, for example, by the force of a previously tensioned drive spring, compressed elastic member or automatic drive such as a motor.

The rotation member reaches its end position at the end of the injection when the dose has been completely injected. The rotation member then abuts a stop surface. At the end of the rotation or near the end the end of injection signal may be produced by the signal member. The rotation member triggers the signal. Preferably, the rotation member may release the signal member from the tensioned state and moves the signal member into a release state thereby generating the end of injection signal.

The tensioning, however, is not triggered by the rotation member but by an exclusive longitudinal relative tensioning movement between a tensioning member and the signal member.

The signal member is flexible or resilient and can be tensioned (prestressed) to store energy for generating an audible and/or tactile signal for the user. The signal member may be, for example, an elastic arm, lever, beam or cam that can be deflected or compressed or otherwise deformed to store energy in the tensioned state.

The signal member may be a separate member or it may be connected to a component or integrally formed (monolithic) in a component of the dose or dispensing mechanism such as an actuation sleeve or dispensing button.

The flexible signal member may be deformable in any direction, for example in a radial direction towards a middle axis or longitudinal axis of the housing or it may be deformable in the longitudinal direction or in a combination thereof.

The signal member can be tensioned and thus be moved from a released state into the tensioned state by the relative tensioning movement between the tensioning member and the signal member. The tensioning member may be movable relative to the housing and the signal member may remain stationary. Alternatively, the signal member may be movable relative to the housing and the tensioning member may be fixed and non-movable relative to the housing.

During the tensioning movement the signal member and the tensioning member hit and contact each other causing the flexible signal member to be deformed such that it is brought into its tensioning state.

The tensioning member may be free to move relative to the housing, for example, if it is connected to a movable component of the dose or dispensing mechanism of the injection device. Alternatively, the tensioning member may be fixed to the housing or may be an integrally formed in the housing (monolithic).

The tensioning member may include a contact surface to facilitate deflection or compression or deformation of the flexible signal member upon relative tensioning movement.

The tensioning member may be rigid, or alternatively it may be flexible and hence it may be deformable during the tensioning movement. In this case, the tensioned signal member may be released by releasing or deforming the tensioning member.

The injection device is preferably a pen-shaped injection device. In a preferred embodiment the injection device is a disposable injection device. Alternatively, the injection device may be a reusable or semi-reusable device. A semi-disposable injection device comprises components or sub-assemblies that are disposed of after an injection or once the medication is fully dispensed. Additionally, it comprises further components or sub-assemblies that can be reused (reusable part) for several injections and successively with several identical disposable parts.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The signal member may be directly coupled or indirectly coupled via intermediate member to a button. The button may be pressed or actuated by the user to start the injection. The button is movable along the longitudinal axis, preferably exclusively movable longitudinally without rotation to provide the relative tensioning movement to bring the signal member into the tensioned state.

Hence, the signal member may be tensioned by the user force when the user actuates the button or when the user releases the button.

The signal member may be integrally formed in the button as monolithic part or alternatively the signal member may be fixedly (rotationally and longitudinally) connected to the button.

The tensioning member may be fixed to the housing such that it does not move when the button is actuated such that the button and the signal member move relative to the tensioning member and thereby engage and deform and thus tension the signal member.

Alternatively, the signal member may be connected to another longitudinally moving component of the injection device such as, for example, an actuation sleeve.

The signal member may include a contact edge, or a contact surface adapted to hit and contact a surface of the tensioning member to deflect, deform or compress the signal member during the relative tensioning movement. The tensioning member may include a sloped surface adapted to engage the contact edge or contact surface of the signal member. Upon contact the signal member may be deformed (e.g. deflected or compressed) such that it moves from the release state into the tensioned state.

The sloped surface of the tensioning member may facilitate the deforming and thus the tensioning of the signal member. In particular, the sloped surface may help to avoid a misalignment or blocking of the flexible signal member.

In a preferred embodiment an angle between a plane perpendicular to the longitudinal axis and the sloped surface is between 45° and 70° and more preferably the angle is about 50°. These angles allow an easy and optimal deformation of the signal member during the relative tensioning movement.

In preferred embodiment, the tensioning member may include a second sloped surface in addition to the above-mentioned sloped surface. The second sloped surface may be adapted to guide the signal member during movement from the tensioned state into the released state. The second sloped surface may help to ensure that the signal member correctly generates the audible or tactile end of injection signal. Namely, the second sloped surface may avoid any misalignment or blocking of the signal member.

The first sloped surface may be proximally oriented, and the second sloped surface may be distally oriented. Preferably, an angle between a plane perpendicular to the longitudinal axis and the distally oriented second sloped surface is preferably between 50° and 70° and more preferably about 60°.

The signal member may be deflected radially inwards when moved from the released state to the prestressed state by the relative tensioning movement. That means the signal member is movable mainly in a plane perpendicular to the longitudinal axis and hence is radially movable. This may enable a reliably tensioning of the signal member. Preferably, the signal member is movable radially back into the release state to generate the end of injection signal.

Accordingly, the tensioning member may include a holding surface adapted to hold the tensioned signal member in the tensioned state. The holding surface is preferably oriented parallel to the longitudinal axis or tangential to a plane parallel to the longitudinal axis. As the signal member moves radially the holding surface may reliably keep the signal member in the biased state prior generation of the end of injection signal.

In this embodiment, the holding surface and thus the tensioning member may be part of the housing or fixedly connected to the housing.

Preferably, the signal member is movable from the tensioned state to the released state in a radial direction and hence deflects radially back into an initial or starting position.

The signal member may include a signal surface on an outer surface or outermost surface adapted to abut a counter surface to generate the end of injection signal. The counter surface is preferably on the tensioning member or on an inside of the housing.

The signal surface ensures that the signal member contacts and engages the counter surface in a predefined manner and thus ensures a reliable generation of the end of injection signal.

In an alternative embodiment, the signal member may be deflected in a direction along longitudinal axis. Also, in this embodiment the tensioned signal member may be released by a rotation of the rotating member towards the end position.

The rotation member may directly release the signal member or the signal member may be indirectly released by a deflection of the tensioning member. The latter in turn may be deflected by the rotating rotation member.

The signal member may be an arm or lever-shaped element with a free end and an end connected to a basis, for example to a sleeve or button of the injection device. The signal member in form of an arm may easily deflected and can thus easily be brought from the release state into the tensioned state and back to the released state. The arm may extend in a plane perpendicular to the longitudinal axis or alternatively the arm may extend along the longitudinal direction.

Alternatively, the signal member may be a cam or knob-shaped element that is compressible into the biased state.

The arm may be curved in a circumferential direction and hence in a shape curved around the longitudinal axis in a plane perpendicular to the longitudinal axis such that the arm can be arranged inside a sleeve. That allows for a compact design.

Preferably, the tensioning member includes a protrusion or a longitudinal element extending in a direction perpendicular to the longitudinal axis. The tensioning member may be fixedly coupled to the housing or to a housing insert which is fixed relative to the housing.

The protrusion or longitudinal element facilitates the deformation of the signal member during the tensioning movement and ensures that the signal member is actually in its tensioned state after the tensioning movement.

The rotation member may comprise a trigger surface or a trigger element adapted to release the signal member from the tensioned state during rotation of the rotation member such that the signal member is moved from the tensioned state to the released state to generate the end of injection signal. Hence, the trigger surface or trigger element rotates with the rotation member and hits the signal member and thereby releases the signal member such that it is deflected back or moved back from the tensioned state into the released state and thereby generates the audible or tactile end of injection signal.

The trigger surface may be arranged on an outer surface and preferably on a front surface of the rotation member. The trigger surface or element may be, for example, a cam, protrusion or arm of the rotation member. The trigger element preferably extends or protrudes in the longitudinal direction.

The rotation member may be a number sleeve rotatable during both, dose setting and dose dispensing and comprises dose indications, for example in form of numbers or signs indicating a specific dose to the user.

The number sleeve may be selectively coupled to or decoupled from a dose set knob and/or a drive sleeve of the dispensing mechanism of the injection pen. The number sleeve may be in threaded connection with the housing of the injection device such that the number sleeve rotates during dose setting and dose dispensing.

The invention further relates an injection device comprising further comprising the button, a dose knob, a clutch, a driver and a plunger rod coupled to the driver, preferably permanently and rotationally coupled to the driver. The injection device may be in a dose setting state and in a dose dispensing state. In the dose setting state, when the user sets a dose, the clutch is rotationally coupled to the dose knob and the clutch is decoupled from the driver such that the plunger rod does not move during dose setting and dose correction.

In the dispensing state the clutch is rotationally coupled to the driver and thus to the plunger rod and the clutch is decoupled from the dose knob. The number sleeve is permanently rotationally coupled to the clutch in dose setting state and in the dose dispensing state. The injection device can be switched from the dos setting state to the dose dispensing state by pressing the button in distal direction.

The invention further relates to a method for generating an end of injection click with an injection device for dispensing a dose of a liquid drug, the method comprising the steps of
- Moving a tensioning member in a distal direction and thereby generating a relative movement between a flexible signal member and the tensioning member along the longitudinal axis without rotation;
- Deforming and thereby moving the flexible signal member from a released state into a tensioned state
- Rotating a rotation member relative to a device housing to dispense a dose
- Hitting and releasing the tensioned signal member from the tensioned state to the released state and thereby generating an audible or tactile end of injection signal only at the end of the injection, by the rotation member, when the rotation member reaches an end position.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a sectional view of an injection pen with a signalling mechanism according to the present invention where the cut for the sectional view runs along the longitudinal axis and where the injection pen is in a dose setting state;
- Fig.2: depicts a sectional view of the injection pen in a dose dispensing state;
- Fig.3a: depicts a proximal portion of the injection pen without the outer housing where a flexible click arm of the signaling mechanism is in a released position;
- Fig.3b: depicts a sectional view of the signaling mechanism in the state shown in figure 3a;
- Fig.4a: depicts the click arm in a tensioned position when the button is pressed;
- Fig.4b: depicts a sectional view of the signaling mechanism in the state shown in figure 4a;
- Fig.5: depicts the click arm in a relaxed position after the generation of an end of injection signal and
- Fig.6: depicts a perspective view of a proximal portion of a number sleeve.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

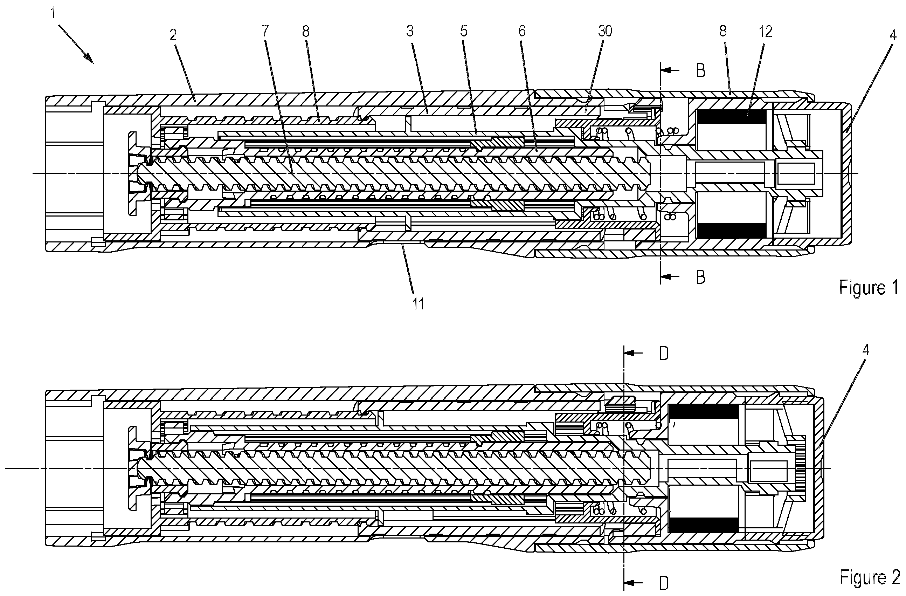

Figures 1 and 2 depict an injection device in form of a reusable spring-driven injection pen 1 which is also named as autopen. Figures 1 and 2 depict a sectional view where the cut for the section runs along the longitudinal axis of the injection pen. Figure 1 depicts the pen in a dose setting state and figure 2 depicts the pen in a dose dispensing state.

The injection pen 1 includes a sleeve-shaped housing 2 accommodating a dose and dispensing mechanism and defining a longitudinal axis. A cartridge holder (not shown) can be attached to the housing to hold a cartridge with the liquid drug. An injection needle may be mounted on a distal end of the cartridge holder. The cartridge holder can be attached or detached from the housing 2 to replace a cartridge as known in the art.

The injection pen 1 includes a sleeve-shaped insert 9 fixed to the housing or integrally formed with the housing. The insert includes an outer thread engaging an inner thread or thread segment of a dose member or number sleeve 3 (also named as indication barrel) which is located coaxially inside the housing 2. The number sleeve 3 indicates dose values which a user can see through an opening or window 11 in the housing 2.

The injection pen 1 further includes a clutch sleeve 5 (or dispensing member), a drive sleeve 6, a dose knob 8, a drive spring 12 and a button 4 that can be pressed in distal direction by the user to start the injection. The button 4 is adapted to selectively couple the clutch sleeve 5 to the dose knob 8 or to couple the clutch sleeve 5 to the drive sleeve 6 for dispensing a dose as will be described in detail below.

The clutch sleeve 5 is coaxially arranged inside the number sleeve 3 and the drive sleeve 6 in turn is located inside the clutch sleeve 5. The clutch sleeve 5 can be selectively, releasably and rotationally coupled to the drive sleeve 6 by a spline engagement. The drive sleeve 6 is permanently rotationally coupled to the plunger rod 7 which is in threaded engagement with the housing 2.

To set a dose the user rotates the dose knob 8 and as the button 4 is in a proximal position the clutch sleeve 5 is rotationally coupled to the dose knob 8 but the clutch sleeve 5 is decoupled from the drive sleeve 6. Rotating the dose knob 8 rotates thus the clutch sleeve 5 and also the number sleeve 3 and additionally biases the drive spring 12. A ratchet prevents unintentional release of the biased spring 12.

Figure 2 depict the pen in a dispensing state with the button in a distal position. To dispense the dose the user presses the button 4 in distal direction. That triggers several operations. First, the clutch sleeve 5 is moved distally along the longitudinal direction and thereby the clutch sleeve 5 gets into engagement with the drive sleeve 6 and is thus rotationally coupled with the drive sleeve 6. Second, the axial movement of the clutch sleeve 5 causes the dose knob 8 to be decoupled from the clutch sleeve 5. The number sleeve 3 remains permanently coupled to the clutch sleeve 5. Third, the clutch sleeve 5 is no longer coupled to the ratchet meaning that it is free to rotate. That means the biased spring 12 can rotate the clutch sleeve 5, the drive sleeve 6 and thus the plunger rod 7 which is screwed in distal direction and dispenses the set dose.

As the number sleeve 3 remains rotationally coupled to the clutch sleeve 5 the number sleeve 3 is rotated during both dose setting and dose dispensing.

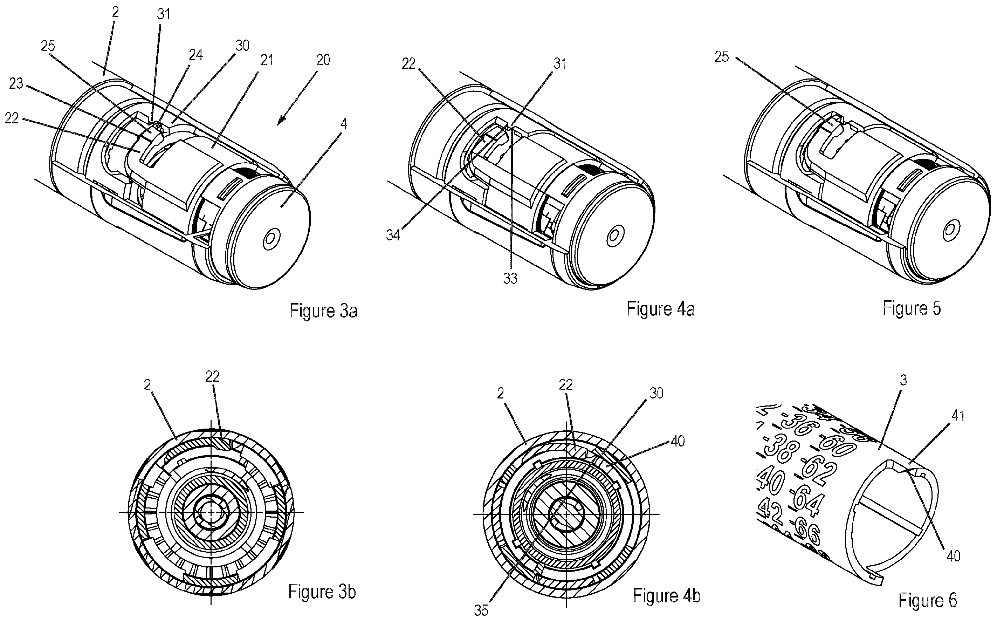

The injection pen further includes an end of injection signaling mechanism 20 which is described in detail in the following with respect to figures 3a, 3b, 4a, 4b and 5. Figures 3a, 4a and 5 depict a proximal end portion of the injection pen without the outer housing and figures 3b and 4b depict a sectional view where the cut for the section runs through the click arm in a plane perpendicular to the longitudinal axis.

The signaling mechanism 20 comprises a sleeve-shaped connector 21 which is axially coupled to the button 4 via intermediate member. The connector moves with the button in distal direction. For this purpose, the connector is axially guided by the housing 2 by splines allowing a movement along the longitudinal axis but preventing the connector 21 from being rotated relative to the housing.

At the distal end the connector 21 includes a flexible signal member or click arm 22 extending in a circumferential direction. The click arm 22 is arc-shaped such that it fits into the housing as best shown in figures 3b and 4b.

The click arm 22 is adapted to be radially deflected from a released state into a radially inner tensioned state. In starting or initial condition and during dose setting and before the user starts the injection and hence before the user presses the button 4 the click arm 22 is in its release state in which the click arm 22 is not tensioned and relaxed. The state is shown in figure 3a and 3b.

On its free end the arm includes an end portion 24 adapted to be moved radially inwards by a protrusion 31 of a tensioning member 30 as described below. The click arm 22 includes further a distally oriented arm sloped surface 23 adapted to engage a first sloped surface 33 (see figure 4a) of the protrusion 31 to facilitate the deflection.

The click arm 22 further includes on a radial outside oriented click surface 25 (figure 3a) adapted to hit or abut an inside of the housing or inside the dose knob 8 to generate a click sound signaling the user the end of the injection.

The tensioning member 30 is sleeve-shaped and fixedly connected to the housing such that it cannot be moved relative to the housing. The tensioning member 30 comprises the protrusion 31 extending in a direction perpendicular to the longitudinal axis and comprising the first sloped surface 33 on a distal side and a second sloped surface 34 on a proximal side as shown in figure 4a. An angle between the first sloped surface 33 and a plane perpendicular to the longitudinal axis and an angle between the second sloped surface 34 and a plane perpendicular to the longitudinal axis is about 50°.

Furthermore, the tensioning member 30 includes a holding surface 35 (figure 4b) adapted to hold the tensioned click arm 22 in the tensioned state as shown in figure 4b. The holding surface is oriented parallel to the longitudinal axis.

In the following the function of the end of injection signalling mechanism is described in detail.

Figures 1, 3a and 3b depict the injection pen 1 in a starting, initial or unused state. That means the button is not pressed and the click member is in a released and relaxed state. As best shown in figure 3b the arc-shaped click arm 22 is not deflected.

When the user presses the button 4 to start the injection the connecter 21 is moved in distal direction by the button. This causes the distally oriented arm sloped surface 23 to get in contact with proximally oriented first sloped surface 33 of the protrusion 31 of the tensioning member 30. As the button 4 is further moved distally the tensioning member 30 and the click arm 22 are moved relative to each other in longitudinal direction. That means the arm sloped surface 23 and the protrusion first sloped surface slide 33 along each other causing the arm 22 to be deflected radially inwards. This causes the arm end portion 24 to get under the holding surface 35 of the tensioning member which means the arm 22 is held deflected by the holding surface. The click arm 22 is then in its tensioned state This state is shown in figure 4a and 4b.

The injection is then started and the pretensioned drive spring 12 is released and the spring force drives the plunger rod 7 in distal direction to dispense the drug. As mentioned above, during dispensing the number sleeve 3 is rotated back towards a start or initial position.

Figure 6 depicts a proximal portion of the number sleeve 3 without other components. In its proximal front end the number sleeve 3 has two circumferential ramp forming two oppositely arranged ledges 41 as shown in figure 6. Each ledge has a proximally oriented sloped trigger surface 40.

Before the number sleeve 3 reaches its initial or start position the sloped trigger surface 40 (figures 4b and 6) on the proximal front of the number sleeve 3 gets into contact with the distally oriented arm sloped surface 23 and in particular with the arm sloped surface 23 thereby facilitating deflection of the click arm 22. As the number sleeve 3 is rotated towards its end position the ledge 41 further moves proximally thereby moving the tensioned click arm 22 out of its tensioned position and away from the holding surface 35 such that it can release. That means the tensioned flexible click arm 22 can relax and thus moves radially outwards until the click surface 25 of the arm hits an inner surface of the housing 2 or of the dose knob 8 thereby generating an end of injection click only at the end of the injection (figure 5). The second sloped surface 34 of the protrusion 31 guides the arm 22 during its relaxation.

The click arm 22 is then in its released state again. When the user releases the button 4 the button and the connector 21 are moved proximally by a button spring force into to start or initial position.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | |
|---|---|---|
| 1 | Injection pen | |
| 2 | housing | |
| 3 | number sleeve | |
| 4 | button | |
| 5 | clutch sleeve | |
| 6 | drive sleeve | |
| 7 | plunger rod | |
| 8 | dose knob | |
| 9 | insert | |
| 11 | window | |
| 12 | drive spring | |
| 20 | end of injection signaling mechanism | |
| 21 | connector | |
| 22 | click arm | |
| 23 | arm sloped surface | |
| 24 | end portion | |
| 25 | click surface | |
| 30 | tensioning member | |
| 31 | protrusion | |
| 33 | first sloped surface | |
| 34 | second sloped surface | |
| 35 | holding surface | |
| 40 | | |
| 40 | trigger surface | |
| 41 | ledge | |

## Claims

1. An end of injection signalling mechanism (20) for an injection device (1) for dispensing a dose of liquid drug through an injection needle, the signalling mechanism (20) comprising a housing (2) defining a longitudinal axis;
a rotation member (3) rotatable relative to the housing (2) during dispensing of the dose towards an end position in which the dose is completely dispensed;
a flexible signal member (22) adapted to generate an audible or tactile end of injection signal only at the end of the dose dispense,
wherein the signal member (22) can be released from a tensioned state by a rotation of the rotation member (3) to generate the signal,
**characterized in that** the signalling mechanism (20) further includes a tensioning member (30) adapted to deform the signal member (22) wherein the signal member (22) can be deformed and tensioned by a relative tensioning movement between the tensioning member (30) and the signal member (22) exclusive along the longitudinal axis without relative rotation.

2. Signalling mechanism (20) according to claim 1, wherein the signal member (22) is directly or indirectly coupled to a button (4) which is movable along the longitudinal axis to provide the tensioning movement.

3. Signalling mechanism (20) according to claim 1 or 2, wherein the tensioning member (30) includes a first sloped surface (33) adapted to engage an edge or surface of the signal member (22) to facilitate deformation of the signal member (22) during the tensioning movement.

4. Signalling mechanism (20) according to claim 3, wherein an angle between the first sloped surface (33) and a plane perpendicular to the longitudinal axis is about between 45 and 70°.

5. Signalling mechanism (20) according to claim 3 or 4, wherein the tensioning member (30) includes a second sloped surface (34) arranged on a opposite side than the first sloped surface, wherein the second sloped surface (34) is adapted to guide the signal member after release from the tensioned state.

6. Signalling mechanism (20) according to any of claims 1 to 5, wherein the signal member (22) is deflected radially inwards or in a direction along the longitudinal axis during the tensioning movement.

7. Signalling mechanism (20) according to claim 6, wherein the tensioning member (30) includes a holding surface (35) adapted to hold the tensioned signal member in the tensioned state and oriented parallel to the longitudinal axis.

8. Signalling mechanism (20) according to any of claims 1 to 7, wherein the signal member (22) is movable from the tensioned state to the released state in a radial direction to abut a surface to generate the end of injection signal.

9. Signalling mechanism (20) according to any of claims 1 to 8, wherein the signal member (22) is an arm or lever-shaped element.

10. Signalling mechanism (20) according to claim 9, wherein the arm is curved in a circumferential direction around the longitudinal axis such that the arm can be arranged inside a sleeve-shaped housing.

11. Signalling mechanism (20) according to any of claims 1 to 10, wherein the tensioning member (30) includes a protrusion (31) or a longitudinal element fixedly coupled to the housing (2).

12. Signalling mechanism (20) according to any of claims 1 to 11, wherein the rotation member (3) comprises a trigger surface or trigger element (40) adapted to hit and to release the signal member (22) from the tensioned state during rotation of the rotation member (3) when the rotation member reaches its end position.

13. Signalling mechanism (20) according to any of claims 1 to 12, wherein the rotation member (3) is a number sleeve rotatable during both, dose setting and dose dispensing and comprising indications representing a dose value.

14. Injection device (1) including a signalling mechanism (20) according to claim 13, and further comprising a button (4), a dose knob (8), a clutch (5), a driver (6) and a plunger rod (7) coupled to the driver (6) and wherein in a dose setting state the clutch (5) is rotationally coupled to the dose knob (8) and the clutch (5) is decoupled from the driver (6) and in a dispensing state the clutch (5) is rotationally coupled to the driver (6) and thus to the plunger rod (7) and decoupled from the dose knob (8) and wherein the number sleeve (3) is permanently rotationally coupled to the clutch (5) in dose setting state and in the dose dispensing state.

15. Method for generating an end of injection click with an injection device (1) for dispensing a dose of a liquid drug, the method comprising the steps of
- Moving a tensioning member (30) in a distal direction and thereby generating a relative movement between a flexible signal member (229 and the tensioning member (30) along the longitudinal axis without rotation;
- Deforming and thereby moving the flexible signal member (22) from a released state into a tensioned state;
- Rotating a rotation member (3) relative to a device housing to dispense a dose;
- Hitting and releasing the tensioned signal member (22) and thereby generating an audible or tactile end of injection signal only at the end of the injection, by the rotation member (3), when the rotation member reaches an end position.
